(19) Europäisches Patentamt / European Patent Office / Office européen des brevets

(11) **EP 2 365 777 B1**

(12) **EUROPÄISCHE PATENTSCHRIFT**

(45) Veröffentlichungstag und Bekanntmachung des Hinweises auf die Patenterteilung:
**07.08.2013 Patentblatt 2013/32**

(21) Anmeldenummer: **10801579.3**

(22) Anmeldetag: **21.12.2010**

(51) Int Cl.:
**A61B 5/00** *(2006.01)*    **A61K 49/00** *(2006.01)*

(86) Internationale Anmeldenummer:
**PCT/EP2010/070408**

(87) Internationale Veröffentlichungsnummer:
**WO 2011/076804 (30.06.2011 Gazette 2011/26)**

(54) **BESTIMMUNG DER LEBERLEISTUNG MITTELS QUANTITATIVER MESSUNG DER METABOLISIERUNG VON SUBSTRATEN**

DETERMINING THE LIVER FUNCTION BY QUANTITATIVELY MEASURING THE METABOLISM OF SUBSTRATES

DÉTERMINATION DE LA FONCTION HÉPATIQUE  PAR MESURE QUANTITATIVE DE LA MÉTABOLISATION DE SUBSTRATS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.12.2009 DE 102009055321**

(43) Veröffentlichungstag der Anmeldung:
**21.09.2011 Patentblatt 2011/38**

(73) Patentinhaber: **Humedics GmbH**
**10625 Berlin (DE)**

(72) Erfinder:
• **HEYNE, Karsten**
**14979 Großbeeren (DE)**
• **STOCKMANN, Martin**
**14129 Berlin (DE)**

(74) Vertreter: **Sokolowski, Fabian**
**Patentanwälte**
**Maikowski & Ninnemann**
**Postfach 15 09 20**
**10671 Berlin (DE)**

(56) Entgegenhaltungen:
**WO-A2-2007/000145**

• **STOCKMANN MARTIN ET AL: "Prediction of postoperative outcome after hepatectomy with a new bedside test for maximal liver function capacity", ANNALS OF SURGERY, J.B. LIPPINCOTT COMPANY, PHILADELPHIA, US, vol. 250, no. 1, 1 July 2009 (2009-07-01), pages 119-125, XP009145009, ISSN: 0003-4932**
• **MARTIN STOCKMANN: "Wertigkeit eines neu entwickelten Verfahrens zur Bestimmung der Leberfunktion in der Leberchirurgie (LiMAx-Test)", 20090201, 1 February 2009 (2009-02-01), pages 1-133, XP007920728,**
• **J. F. LOCK ET AL: 'Interpretation of non-invasive breath tests using 13C-labeled substrates - A preliminary report with 13C-methacetin' EUROPEAN JOURNAL OF MEDICAL RESEARCH Bd. 14, 14 Dezember 2009, Seiten 547 - 550, XP055030308**
• **ZENG WENBING ET AL: "13C-methacetin breath test parameter S for liver diseases diagnosis", SCIENCE IN CHINA. SERIE C: LIFE SCIENCE, GORDON AND BREACH, AMSTERDAM, NL, vol. 39, no. 1, 1 February 1996 (1996-02-01), pages 87-98, XP007920729, ISSN: 1006-9305**

**Beschreibung**

Die Erfindung betrifft ein Verfahren zur Bereitstellung von Ausgangsdaten zur Bestimmung der Leberleistung eines Lebewesens nach dem Oberbegriff des Anspruchs 1.

Die Leber stellt ein für die Funktionstüchtigkeit eines Lebewesens, insbesondere von Menschen, lebenswichtiges Organ dar, da in der Leber viele Substanzen, wie z.B. Medikamente enzymatisch abgebaut werden. Der Substanzabbau wird dabei vorwiegend durch die Familie der Cytochrome, insbesondere in Form der P450-Oxygenasen, katalysiert. Dabei ist seit längerer Zeit bekannt, dass unterschiedliche Cytochrome verschiedene Substanzen metabolisieren. Es ist auch bekannt, dass durch die Messung der Konzentration der metabolisierten Substanzen die Funktionstüchtigkeit der Leber abgeschätzt werden kann.

So wird in einem Artikel von Matsumoto et al. (Digestive Diseases Science, 1987, Vol. 32, Seiten 344 - 348) die orale Verabreichung von $^{13}$C-Methacetin an gesunde und lebergeschädigte Patienten beschrieben, wobei das $^{13}$C-Methacetin in der Leber unter Freisetzung von $^{13}CO_2$ umgesetzt wird. Die Bestimmung des $^{13}CO_2$-Gehaltes in der Ausatemluft ermöglicht damit dabei eine Aussage über den Schädigungsgrad der Leber.

Braden et al. (Aliment Pharmacol. Ther., 2005, Vol. 21, Seiten 179 - 185) beschreibt die Messung des $^{13}CO_2/^{12}CO^2$-Verhältnisses in der Ausatemluft von Individuen, denen ebenfalls zuvor $^{13}$C-Methacetin oral verabreicht wurde. Es wird dabei vorzugsweise über einen Zeitraum von 60 min kontinuierlich gemessen, um die maximale Enzymaktivität zu bestimmen.

Diese Herangehensweise reicht aber für den Einsatz in der klinischen Praxis nicht aus, da sich insbesondere aufgrund der oralen Verabreichung des $^{13}$C-Methacetins lediglich eine Aussage darüber ableiten lässt, ob die Leber funktioniert oder eventuell noch einigermaßen gut funktioniert. Daraus lässt sich jedoch keine direkte Behandlungsstrategie für den Arzt ableiten.

Darüber hinaus sind die bisher angewendeten Verfahren in der Leberdiagnostik nicht individuell spezifisch, sondern erlauben lediglich statistische Aussagen über Patientengesamtheiten. Das bedeutet, dass mittels der erwähnten Messungen Aussagen getroffen werden können, ob bei einem bestimmten Messergebnis die Wahrscheinlichkeit für einen negativen Befund erhöht ist oder nicht. Auch kann von der individuellen Messung nicht direkt auf die Leberleistung geschlossen werden.

Es ist daher wünschenswert, einfache Tests zu entwickeln, die prognostische Aussagen bezüglich der funktionellen Reserven des Leberzellgewebes ermöglichen. Konventionelle Laborparameter sind nicht sensitiv genug, um die komplexen biologischen Vorgänge in der Leber sowie deren Veränderungen bei Erkrankung verlässlich zu evaluieren.

Ein Analyseverfahren, welches eine quantitative Bestimmung der Leberfunktion ermöglicht, ist in der WO 2007/000145 A2 beschrieben. Das Verfahren basiert auf einer Substratanflutung des zu metabolisierenden Substrates in der Leber und der Bestimmung der maximalen Umsatzgeschwindigkeit des Substrates, die Aussagen über die Leberfunktionskapazität eines Patienten ermöglicht.

Ein Verfahren, dass eine individuelle Aussage über die quantitative Metabolisierungsleistung eines einzelnen Organes, insbesondere der Leber, zulässt, kann in verschiedenen Ausgestaltungen folgende Eigenschaften aufweisen:

1.) Die Dynamik der Metabolisierung eines Substrates in der Leber des Patienten wird in Echtzeit und hoch aufgelöst untersucht. Dabei kann vorgesehen sein, dass die Intiierung der Metabolisierung im Vergleich zum Anstieg der Metabolisierung schnell erfolgt, d. h., es ist wünschenswert, wenn 70 % der Intiierung der Metabolisierung mindestens um das 2-fache schneller verläuft.

2.) Die Metabolisierung wird direkt gemessen, d. h., dass entweder ein Metabolisierungsprodukt direkt einer Messung zugänglich ist oder aber eine andere Größe, die in einer festen Proportionalität zum Metabolisierungsprodukt steht, kann direkt gemessen werden. Dies bedeutet z. B. bei Atemgasuntersuchungen, dass vorzugsweise jeder Atemzug, zu mindestens aber zwei Atemzüge pro Minute gemessen wird. Es wird also auf eine Zwischenlagerung der Atemgasprobe und eine Teilentnahme aus dem Atemgas mit Hinblick auf die möglichen auftretenden Verfahrensfehler verzichtet.

3.) Die gemessene Größe verändert sich nicht um mehr als 20 % durch physiologische Faktoren, d. h. je geringer der Einfluss von physiologischen Faktoren ist, wie z. B. die Verteilung des Substrates im Körper durch das Blut, desto genauer ist die quantitative Bestimmung des Metabolisierungsproduktes.

4.) Der Metabolisierungsprozess des verabreichten Substrates ist eindeutig und läuft ausschließlich oder zu über 90 % in den Leberzellen ab und nirgendwo sonst im Körper.

5.) Der Metabolisierungsprozess variiert in seiner Reaktionseffizienz nicht von Mensch zu Mensch, da dies einer individuellen quantitativen Bestimmung zuwiderlaufen würde. Somit sind Metabolisierungsprozesse, die eine starke genetische Variation aufweisen, ausgeschlossen. Wenn überhaupt, sollte bei einer genetischen Variation des Metabolisierungsprozesses zu mindestens die Stärke der Variation eines genetisch unveränderten Metabolisierungsprozesses bekannt sein.

6.) Es ist am günstigsten, wenn der Metabolisierungsprozess durch Leberenzyme bzw. Lebercoenzyme erfolgt, die gleichmäßig in allen Leberzellen der Leber auftreten. Tritt eine Häufung von Leberenzymen bzw. Lebercoenzymen in spezifischen Bereichen der Leber auf, kann maximal eine für diese Bereiche gewichtete Aussage über die Leberleistung getroffen werden. Auch dürfen die Leberenzyme bzw. Lebercoenzyme nicht durch andere Metabolisierungsreaktionen so stark belastet werden, dass dies zu einer Änderung des Metabolisierungsprozesses in einer Größenordnung von mehr als 30% und damit einer um mehr als 30%igen Änderung der Metabolisierungsdynamik führt.

Mit den bisher bekannten Verfahren ist es nicht möglich, die angeführten Punkte zu verwirklichen.

Der vorliegenden Erfindung liegt die Aufgabe zugrunde, ein Verfahren bereitzustellen, welches eine individuelle Aussage über die quantitative Metabolisierungsleistung der Leber zulässt.

Diese Aufgabe wird durch das vorliegende Verfahren zur Bereitstellung von Ausgangsdaten zur Bestimmung der Leberleistung eines Lebewesens, insbesondere der Leberleistung eines Menschen, gemäß Anspruch 1 gelöst.

Dabei umfasst das erfindungsgemäße Verfahren den Schritt der Bestimmung des Gehaltes des gebildeten $^{13}CO_2$ als Metabolisierungsprodukt eines mit $^{13}C$-markierten Substrats in der Ausatemluft über ein bestimmtes Zeitintervall mittels eines Messgerätes mit mindestens einer Auswerteeinheit, wobei das Substrat dem Lebewesen zuror verabreicht wurde und durch die leber unter Freisetzung des $^{13}CO_2$ ungesetzt wird. Die Menge des gebildeten $^{13}CO_2$ in der Ausatemluft ist dabei bevorzugt proportional zu der Menge des mindestens einen verabreichten Substrates. Das erfindungsgemäße Verfahren ist dadurch charakterisiert, dass basierend auf den ermittelten Messpunkten die gemessene anfängliche Zunahme des Gehaltes des $^{13}CO_2$ in der Ausatemluft mit einer Differentialgleichung erster Ordnung beschrieben wird. Aus der Lösung dieser Differentialgleichung erster Ordnung werden folgend ein maximaler Wert $A_{max}$ (auch als $DOB_{max}$ bezeichnet, wobei DOB für "delta over baseline" steht) sowie eine Zeitkonstante tau der Zunahme des $^{13}CO_2$-Gehaltes bestimmt.

Der maximale Wert $A_{max}$ bzw. $DOB_{max}$ entspricht dabei dem Maximum der Metabolisierungsdynamik und die Zeitkonstante tau entspricht der Zeitkonstante des Anstiegs der Metabolisierungsdynamik. Die Erfindung erlaubt die Anpassung (sogenanntes "Fitten") einer Kurve an die tatsächlich gemessenen Werte der zeitlichen Änderung des $^{13}C$-Gehalts, wobei diese Kurve eine Lösung der Differentialgleichung erster Ordnung darstellt und mindestens zwei Werte ausweist, nämlich den Maximalwert $A_{max}$ und die Zeitkonstante $\tau$ (tau). Insbesondere handelt es sich bei der Lösung der Differentialgleichung um eine Exponentialfunktion, die die anfängliche Zunahme des Gehaltes des $^{13}CO_2$ in der Ausatemluft näherungsweise beschreibt. Deren Werte $A_{max}$ und tau stellen charakteristische Größen dar, die das Anfangsverhalten der Zunahme charakterisieren. Damit erlaubt die vorliegende Erfindung durch die Bestimmung zweier Parameter der gemessenen anfänglichen Zunahme eine besonders feingliedrige und hochaufgelöste Analyse von Krankheitsbilder der Leber. Insbesondere erlaubt die Auswertung auch des Parameters tau neben dem Maximalwert $A_{max}$ eine solche feingliedrige Auswertung. Die vorliegende Erfindung stellt damit einem Arzt verbesserte Ausgangsdaten für eine Diagnose bereit.

Das zu metabolisierende Substrat wird in die Leberzellen transportiert. Die Differentialgleichung, mit der der Transport der Substanz in die Leberzellen gelangt, kann durch folgende Gleichung beschrieben werden

$$\frac{d}{dt}X = f(X,Y,Z,..) + C\frac{\partial^2}{\partial z^2}X$$

oder in drei Dimensionen

$$\frac{d}{dt}X = f(X,Y,Z,..) + C\Delta X$$

wobei X die Konzentration des zu metabolisierenden Substrates und C den Diffusionskoeffizient darstellt.

Es wird der Diffusionskoeffizient C in erster Näherung als ortsunabhängig angenommen. Da bei der Auswertung der Metabolisierungsdynamik keine ortsspezifische Auflösung durchgeführt werden kann, bzw. über alle Orte gemittelt wird, reduziert sich die Ortsabhängigkeit auf eine apparente Diffusionskonstante $C_{ava}$ und es ergibt sich die folgende Gleichung:

$$\frac{d}{dt}X = f(X,Y,Z,..) - C_{ave}X$$

Wesentlich ist, dass der Metabolisierungsschritt am Enzym schnell gegenüber der Diffusionsdynamik, d.h. mindestens doppelt so schnell, verläuft. So findet die Metabolisierung z.B. durch das Cytochrom CYP P450 1A2 durchschnittlich im Sub-Millisekundenbereich statt.

Bedingt durch die Metabolisierung des Substrates wird das Substrat von der Leber aufgenommen, wodurch die Substratkonzentration X gesenkt und ein Konzentrationsgradient zwischen Zellinnerem und Zelläußerem aufrecht gehalten wird, bis die Substanz vollständig abgebaut ist.

Faktoren auf einer längerer Zeitskala sind durch die Funktion f(X,Y,Z,...) gegeben. Diese Einflussfaktoren müssen zu Beginn der Metabolisierungsdynamik weniger als 20% der Metabolisierungsdynamik ausmachen, damit die Differentialgleichung (DGL) mit einer DGL erster Ordnung gemäß folgender Gleichung beschrieben werden kann:

$$\frac{d}{dt}X = -C_{ave}X$$

Die Lösung dieser DGL entspricht der Gleichung

$$X(t) = X_0 \exp(-t/C_{ave}),$$

wobei $C_{ava}$ eine Zeitkonstante tau der Umsetzung und X die Konzentration des verabreichten Substrates darstellt.

Der Zeitpunkt t=0 ergibt sich aus der Anpassung der Dynamik oder der Initiierung der Metabolisierung. Wird $^{13}CO_2$ als $^{13}$C-markiertes Metabolisierungsprodukt nachgewiesen, so ist die Zunahme der Konzentration dieses Metabolisierungsproduktes A proportional zur Abnahme des verabreichten Substrates X. Dadurch wird aus dem exponentiell abfallenden Verlauf des Substrates ein exponentiell ansteigender Verlauf des Metabolisierungsproduktes gemäß

$$y(t) = A_{max} - A \cdot \exp(-t/tau),$$

wobei $A_{max}$ die maximale Amplitude der gefitteten Funktion ist und damit für die maximale Konzentration oder Menge des $^{13}CO_2$ steht und tau die Zeitkonstante der Umsetzung ist. Es liegt also eine Exponentialkurve vor, die die Zunahme beschreibt.

Erfindungsgemäß entspricht die Lösung der Differentialgleichung erster Ordnung der Gleichung

$$y(t) = A_{max} - A_0 \exp(-\frac{t - t_0}{tau})$$

wobei y(t) für die Metabolisierungsdynamik des mindestens einen Substrates, t für die Messzeit steht, $t_0$ für den Beginn der Metabolisierung, tau für die Zeitkonstante der Umsetzung und $A_{max}$ für die maximale Amplitude der gefitteten Funktion bzw. die maximale Konzentration

des $^{13}CO_2$ und $A_0$ für die Anfangskonzentration des $^{13}CO_2$ steht.

Aus der obigen Gleichung ist somit eine Bestimmung von $A_{max}$ und der Zeitkonstante tau möglich.

Die genannte Exponentialfunktion wird also bevorzugt an die Messwerte der anfänglichen Zunahme des Gehaltes des $^{13}CO_2$ in der Ausatemluft angepasst. Aus der Anpassung werden dann der maximale Wert $A_{max}$ und die Zeitkonstante tau abgeleitet.

Zur Bestimmung der quantitativen Leberleistung eines Lebewesens ist es dabei von Bedeutung, dass der Wert $A_{max}$ proportional zur Anzahl der an der Metabolisierung beteiligten Leberzellen ist, und dass die Zeitkonstante tau Informationen über die Zugänglichkeit des zu metabolisierenden Stoffes zu den Leberenzymen bzw. Lebercoenzymen gibt.

In einer Ausführungsform der vorliegenden Erfindung wird die $^{13}CO_2$-Zunahme in der Ausatemluft bis zu einem Wert von 70% des Maximalwerts der $^{13}CO_2$-Zunahme bevorzugt bis zum Maximalwert der $^{13}CO_2$-Zunahme mit einer Differentialgleichung erster Ordnung beschrieben.

In einer besonders bevorzugten Ausgestaltung der Erfindung ist es nunmehr möglich, aus dem Wert $A_{max}$ bzw. $DOB_{max}$ das Umsatzmaximum des mindestens einen Substrates in der Leber durch die folgende Gleichung

$$LiMAx = \frac{DOB_{max} R_{PBD} PM}{BW}$$

zu bestimmen, wobei $R_{PDB}$ dem Wert 0,011237 (Pee-Dee-Belemnite-Standard des $^{13}CO_2/^{12}CO_2$-Verhältnisses), P der $CO_2$-Produktionsrate, M der molaren Masse der verabreichten Substanz und BW dem Körpergewicht der Person entsprechen.

Bei Verwendung des erfindungsgemäßen Verfahrens zur Bestimmung der Leberleistung ist dabei zu beachten, dass bei einer großen Zeitkonstante tau das direkt ablesbare Maximum des Metabolisierungsprozesses bzw. der Metabolisierungsdynamik von dem aus der Differentialgleichung erster Ordnung bestimmten Maximums $A_{max}$ bzw. $DOB_{max}$ abweichen kann. Dies liegt darin begründet, dass bei einem langsamen Anstieg der Metabolisierungsrate die Einflüsse anderer Faktoren, wie z. B. die Verteilung des Substrates im Körper, zunehmen. Aus diesem Grunde ist es wünschenswert, die Initiierung der Metabolisierung schnell zu bewirken, wie dies z. B. durch die intravenöse Verabreichung des zu metabolisierenden Substrates erfolgen kann. Die intravenöse Verabreichung des Substrates garantiert eine schnelle Bereitstellung des Substrates in der Leber und die damit verbundene rasche Initiierung der Metabolisierung des Substrates. Auch erlaubt die intravenöse Verabreichung die Bereitstellung eines hinreichend hohen Substratgradienten zwischen den Leberzellen und dem Blut, die die Aufnahme einer Metabolisierungsdynamik und das Erreichen einer maximalen Umsatzgeschwindigkeit des Substrates ermöglicht.

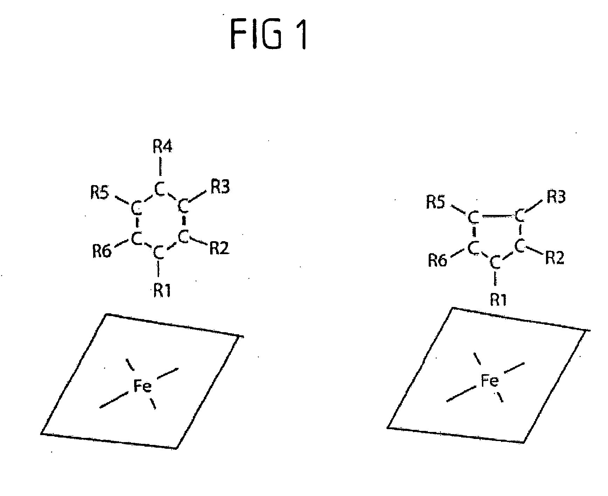

Es ist weiterhin bevorzugt, dass das zu metabolisierende Substrat Struktureinheiten aufweist, die den in der Figur 1 gezeigten Strukturen entsprechen. Insbesondere sollte als $^{13}C$-markiertes Substrat eine Verbindung verwendet werden, die die Freisetzung von $^{13}CO_2$ mittels einer Dealkylisierungsreaktion einer Alkoxygruppe R1, insbesondere einer Methoxygruppe, erlaubt. Im Allgemeinen können die verwendeten Substrate große oder kleine Moleküle sein, die entweder einen Sechsring aus Kohlenstoffatomen oder Kohlenstoffisotopen sowie eine Alkoxygruppe aufweisen, wobei die Alkoxygruppe durch die in der Leber vorhandenen P450-Cytochrome zunächst hydroxyliert werden, wobei sich anschließend $^{13}CO_2$ abspaltet. Beispiele für geeignete Substrate sind unter anderem das $^{13}C$-Methacetin, Phenancetin, Ethoxycoumarin, Koffein, Erythromycin und/ oder Aminopyrin. Dabei ist es auch vorstellbar, dass ein Kohlenstoffatom durch ein anderes Atom, wie z.B. Stickstoff oder Schwefel, ersetzt werden kann. Auch ist es vorstellbar, dass die verwendeten Substrate auf Verbindungen mit einem Fünfring basieren, die mindestens mit einer Alkoxygruppe R1 substituiert sind. Selbstverständlich können auch hier ein oder zwei Kohlenstoffatome des Fünfringes durch andere Atome, wie z. B. Stickstoff oder Schwefel, ersetzt werden. Auch ist es selbstverständlich möglich, dass die verwendeten Substrate verschiedenste Substituenten aufweisen. So können die in der Figur 1 gezeigten Reste R2, R3, R4, R5 und R6 ausgewählt sein aus einer Gruppe enthaltend Halogene, Alkylgruppen, Carboxylgruppen, Estergruppen oder Silangruppen. Selbstverständlich ist diese Aufzählung an möglichen Substituenten nicht abschließend, sondern kann sich auf für den Fachmann geläufige Substituenten erstrecken.

Das $^{13}C$-markierte Substrat wird bevorzugter Weise in einer Konzentration zwischen 0,1 und 10 mg/kg Körpergewicht verabreicht. Die Konzentration des zu metabolisierenden Substrates sollte dabei so ausgewählt sein, dass die Metabolisierungsdynamik im linearen Bereich von der Sättigung entfernt ist. Übersteigt die Substratkonzentration einen bestimmten Wert, ist es nicht mehr möglich, die $^{13}CO_2$-Zunahme in der Ausatemluft mittels einer Differentialgleichung erster Ordnung zu beschreiben. So sollte im Falle der Verwendung von $^{13}C$-Methacetin als zu metabolisierendes Substrat die verabreichte Menge nicht über 10 mg/ kg Körpergewicht liegen.

Im Rahmen des vorliegenden Verfahrens wird bevorzugt der absolute $^{13}CO_2$-Gehalt in der Ausatemluft bestimmt. Dabei sollte die Messung des $^{13}CO_2$-Gehaltes in der Ausatemluft sowohl in Echtzeit als auch kontinuierlich erfolgen. Eine kontinuierliche Bestimmung der Konzentration der $^{13}CO_2$-Konzentration in der Ausatemluft im Messgerät resultiert in der Ermittlung von mehr Datenpunkten, wodurch eine höhere Auflösung und Präzision der aus den ermittelten Datenpunkten gebildeten Messkurve folgt. Eine zuverlässige Bestimmung des maximalen Wertes $A_{max}$ bzw. $DOB_{max}$ und der Zeitkonstante tau sollte auf mindestens fünf Messpunkten, bevorzugt mindestens sieben Messpunkten, basieren.

In einer besonders bevorzugten Ausführungsform wird das vorliegende Verfahren mit weiteren analytischen Verfahren, insbesondere mit der CT-Volumetrie, kombiniert. Dies ermöglicht eine umfassende Aussage über den Gesundheitszustand eines Patienten und eine gezielte Operationsplanung, z.B. im Fall des Auftretens von Tumoren.

In einer weiteren Ausführungsform wird das vorliegende Verfahren mit weiteren analytischen Verfahren, insbesondere der Magnetresonanztomographie (MRT) kombiniert. Dabei wird das zu metabolisierende $^{13}C$-markierte Substrat mit der bildgebenden MRT in der Leber lokalisiert. Die Metabolisierungsdynamik wird durch das vorliegende Verfahren bestimmt und kann mit zeitaufgelöster MRT verglichen werden. Die Kombination beider Verfahren erlaubt es, eine räumliche und zeitliche Auflösung der Metabolisierung einzelner Enzyme insbesondere in der Leber zu untersuchen. Im allgemeinen ist die zeitliche Auflösung der MRT zu langsam um eine Metabolisierungsdynamik zu verfolgen. Synchronisiert man die Daten der Bildgebung jedoch mit der Metabolisierungsdynamik des vorliegenden Verfahrens, so kann ein besseres Abbild der Metabolisierung, z. B. durch Gewichtung der MRT-Daten zu verschiedenen Zeitpunkten, erreicht werden.

Zusätzlich können in einer Variante die zu metabolisierenden $^{13}C$-markierten Substrate so ausgewählt werden, dass sie von Enzymen oder Coenzymen in der Leber metabolisiert werden, die nicht homogen in der gesamten Leber verteilt sind, sondern in spezifischen Regionen verstärkt vorkommen. Dadurch kann die Metabolisierungsleistung einzelner Regionen in der Leber bestimmt werden.

Für die Bestimmung der Metabolisierungsdynamik und der räumlichen Abbildung dieses Prozesses für ein homogen in der Leber verteiltes Enzym oder Coenzym ist zu gewährleisten, dass das Substrat sehr schnell und effizient in die Leberzellen gelangt und dort störungsfrei mittels MRT nachgewiesen werden kann, während gleichzeitig mit dem vorliegenden Verfahren die Metabolisierungsdynamik gemessen wird.

Ein Ausführungsbeispiel hierzu ist das $^{13}C$-markierte Methacetin, dass durch den Lösungsvermittler Propylenglykol in wässriger Lösung in einer ausreichend hohen Konzentration lösbar ist. Die Konzentration des Propylenglykols beträgt 10 bis 100 mg/ml, womit eine Methacetin-Lösung mit einer Konzentration von 0,2 bis 0,6 % Methacetin erreicht werden kann. Diese besondere Kombination von $^{13}C$-markiertem Substrat (Methacetin) und dem Lösungsvermittler Propylenglykol in wässriger Lösung erlaubt eine nahezu untergrundfreie MRT-Messung des $^{13}C$-markierten Methacetins. Das natürli-

che Isotopenverhältnis von $^{13}$C kann die MRT-Messungen stark negativ beeinflussen. Beiträge zu einem stark störenden Untergrundsignal können alle Kohlenstoffatome des Methacetins, des Lösungsvermittlers und der restlichen organischen Substanzen in den Leberzellen liefern. Durch die spezielle Wahl der $^{13}$C-Markierung an der über eine Ethergruppe gebundenen Methylgruppe im Methacetin (also der Methoxygruppe) unterscheidet sich der Isotopenshift des $^{13}$C-markierten Kohlenstoffs in Methacetin von den MRT-Signalen der Kohlenstoffatome des Lösungsvermittlers und der Aminosäuren und somit von den meisten anderen organischen Substanzen in den Leberzellen. Andere Stellen der $^{13}$C-Markierung zeigen diesen Vorteil nicht und verhindern damit nutzbringende MRT-Messungen. Der Kontrast der MRT-Bildgebung kann durch geschickte Wahl der Pulse durch Ausnutzung von Kopplungseffekten (z.B. NOE, DEPT, etc.) erhöht werden.

Insbesondere bei sehr schlechter Leberleistungen bietet die Kombination beider Verfahren deutliche Synergieeffekte. Zusätzlich ermöglicht die Kombination eine räumliche Auflösung der Mikrozirkulation in der Leber.

Die mittels des vorliegenden Verfahrens bestimmten Werte $A_{max}$ und tau können für eine Vielzahl von Anwendungen eingesetzt werden. Folgende Verwendungen und Einsatzgebiete sind dabei von besonderer Bedeutung: Bestimmung der Leberleistung, Verfolgung der Lebergeneration nach einer Operation, Planung von Operationen, insbesondere an einer geschädigten Leber, Bestimmung der Funktion einer transplantierten Leber, Sepsiseinschätzung, insbesondere von Intensivpatienten, Bestimmung der Leberschädigung durch Arzneimittel bei der Arzneinmittelzulassung, Verfolgung von Langzeitschäden der Leber, Bestimmung von Leberschäden durch genetisch veränderte Lebensmittel, auf dem Gebiet der Arbeitssicherheit in der chemischen Industrie, in der Arbeitsmedizin, Leberkrebsvorsorgeuntersuchung, Überwachung von Leberkrankheiten, Einstellung der Dosierung von Arzneimitteln, Nachweis von Leberschäden bei Tieren, in der Umweltmedizin und Routineuntersuchung der Leberfunktion verwendet werden.

Die vorliegende Erfindung soll im Folgenden anhand der nachfolgenden Ausführungsbeispiele unter Bezugnahme auf die Figuren erläutert werden, ohne dass diese Erläuterungen eine einschränkende Wirkung auf den Schutzbereich der Erfindung haben.

Es zeigen:

Figur 1    eine schematische Darstellung der zur Durchführung der Verfahrens geeigneten Substanzen;

Figur 2    eine schematische Darstellung des Ablaufes des Messverfahrens;

Figur 3    eine graphische Darstellung der Anstiegskinetik anhand der gemessenen DOB Werte über die Messzeit;

Figur 4a    eine graphische Darstellung der Anstiegskinetik bei normaler Leberleistung;

Figur 4b    eine graphische Darstellung der Anstiegskinetik bei Leberzirrhose;

Figur 4c    eine graphische Darstellung der Anstiegskinetik bei schweren Leberschäden;

Figur 4d    eine graphische Darstellung der Anstiegskinetik bei Leberversagen;

Figur 5    eine graphische Darstellung des Umsatzmaximums LiMAx über die Zeit bei normaler Leberleistung, eingeschränkter Leberleistung und Leberversagen.

Figur 6    eine schematische Darstellung des Transportes einer verabreichten Substanz in die Leber

Figur 7    eine graphische Darstellung der Anstiegskinetik zur Bestimmung der Daten des maximalen Wertes A und der Zeitkonstante tau

Figur 8    eine graphische Darstellung der Abnahme der Konzentration eines zu metabolisierenden Substrates und der Zunahme der Konzentration eines Metabolisierungsproduktes im Blut

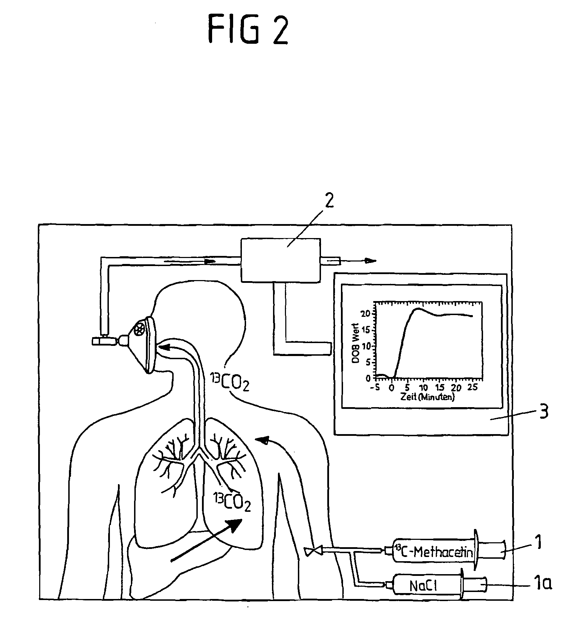

Die Bestimmung der Leberleistung eines Menschen erfolgt bevorzugt einem Schema wie in Figur 2 gezeigt. Bei diesem Messablauf wird die Metabolisierung durch die intravenöse Verabreichung des zu metabolisierenden Substrates, insbesondere $^{13}$C-Methacetin 1 in Verbindung mit isotonischer Kochsalzlösung 1a, gestartet.

Durch die intravenöse Substratverabreichung wird die zur Auswertung erforderliche schnelle Substratanflutung und die rasche Initiierung mit der Substratmetabolisierung gewährleistet. Die Initiierung der Substratmetabolisierung, hervorgerufen durch die enzymatische Umsetzung des Substrates in der Leber ist dabei schneller als der Atemrhythmus.

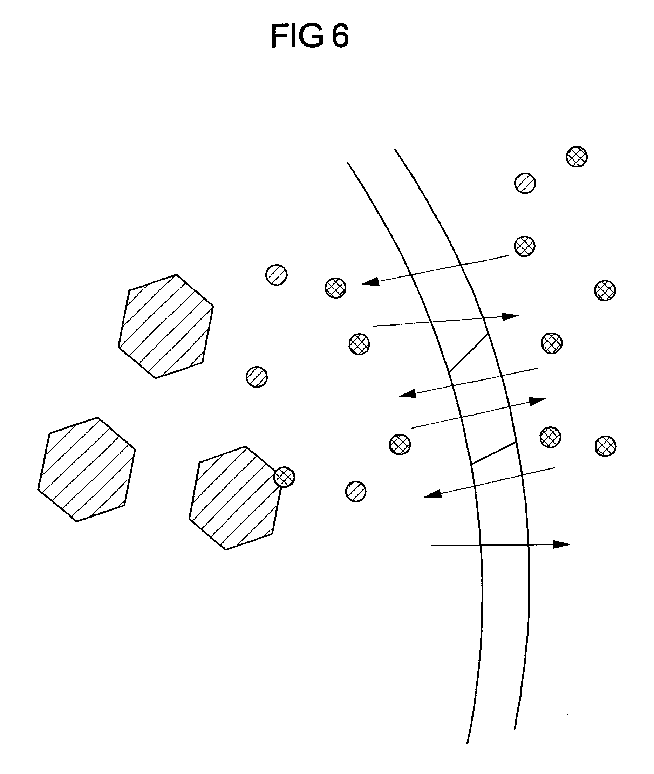

Der Transport des verabreichten Substrates in die Leber und die dort stattfindende Umsetzung bzw. Abbau des Substrates ist schematisch in Figur 6 verdeutlicht. Das verabreichte Substrat (doppelt schraffierte Kreise), wie z.B. $^{13}$C-Methacetin, wird mit einer spezifischen Transportkonstante in die Leberzellen transportiert, dort durch die entsprechenden Enzyme (einfach schraffierte Sechsecke), insbesondere P450 Oxygenasen z.B. mittels Dealkylierung mit einer bestimmten Reaktionskonstante umgesetzt und das dealkylierte Produkt (einfach schraffierte Kreise) z.B. Paracetamol mit einer spezifischen Transportkonstante sowie das $^{13}$C-markierte Metabolisierungsprodukt (einfach schraffierte Kreise) z.B. $^{13}CO_2$ mit einer spezifischen Transportkonstante aus den Leberzellen in das Blut transportiert.

Neben einer enzymatischen Aktivierung des Substrates insbesondere durch P450-Oxygenasen ist auch eine Freisetzung oder Aktivierung des Substrates mittels Belichtung oder weiterer schneller Prozesse vorstellbar. Das freigesetzte Metabolisierungsprodukt, z.B. $^{13}CO_2$ wird über das Blut in die Lunge transportiert und dort abgeatmet. Die Ausatemluft wird bevorzugt über eine Atemmaske und eine Verbindungsleitung kontinuierlich in das Messgerät 2 geleitet und mittels eines Computers 3 ausgewertet (Stockmann et al., Annals of Surgery, 2009, 250: 119-125). Ein für das vorliegende Verfahren einsetzbares Messgerät ist u.a. in der WO 2007/107366 A1 beschrieben.

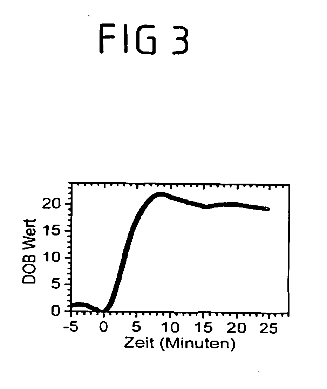

Aufgrund des verwendeten spezifischen Messgerätes ist es möglich, die Metabolisierung des Substrates in jedem Atemzug in Echtzeit zu verfolgen. Dies ist in Figur 3 verdeutlicht. Das Diagramm der Figur 3 zeigt einen Anstieg der $^{13}CO_2$-Konzentration in Form des DOB-Wertes in der Ausatemluft, wobei der Anstieg einer Differentialgleichung erster Ordnung entspricht. Dabei bezeichnet 1 DOB eine Änderung des $^{13}CO_2$-zum-$^{12}CO_2$-Verhältnisses um ein Tausendstel über dem natürlichen Verhältnis. Aus diesem Anstieg sind, wie zuvor beschrieben, sowohl $A_{max}$ bzw. $DOB_{max}$ als auch die Zeitkonstante tau ableitbar. Nachdem die $^{13}CO_2$-Zunahme ein Maximum erreicht hat, erfolgt eine Abnahme der $^{13}CO_2$-Konzentration, was auf weitere dynamische Prozesse im Körper zurückzuführen ist, die zum Abbau des Messsignales beitragen.

Mit der beschriebenen Metabolisierungsdynamik ist es möglich, direkt und unmittelbar die Metabolisierung eines verabreichten Substrates durch die in der Leber vorhandenen Enzyme zu verfolgen. So wird das bevorzugte verabreichte Substrat Methacetin über das Enzym CYP1A2 demethyliert. Durch die Auswertung der Anstiegskinetik des verabreichten Methacetins, die einer Differenzialgleichung erster Ordnung entspricht, und die daraus abgeleiteten Parameter $A_{max}$ und tau ist es nunmehr möglich, die Leberleistung direkt zu bestimmen. Dabei ermöglicht der Maximumwert $A_{max}$ eine Aussage über die Anzahl der gesunden Leberzellen und das damit zur Verstoffwechselung zur Verfügung stehende Lebervolumen, während der Anstieg in Form der Zeitkonstante tau Aussagen über die Eintrittsgeschwindigkeit des Substrates in die Leberzelle ermöglicht. Insbesondere die Zeitkonstante tau ermöglicht also Aussagen darüber, ob die Leber überhaupt in der Lage ist, Substrate aufzunehmen.

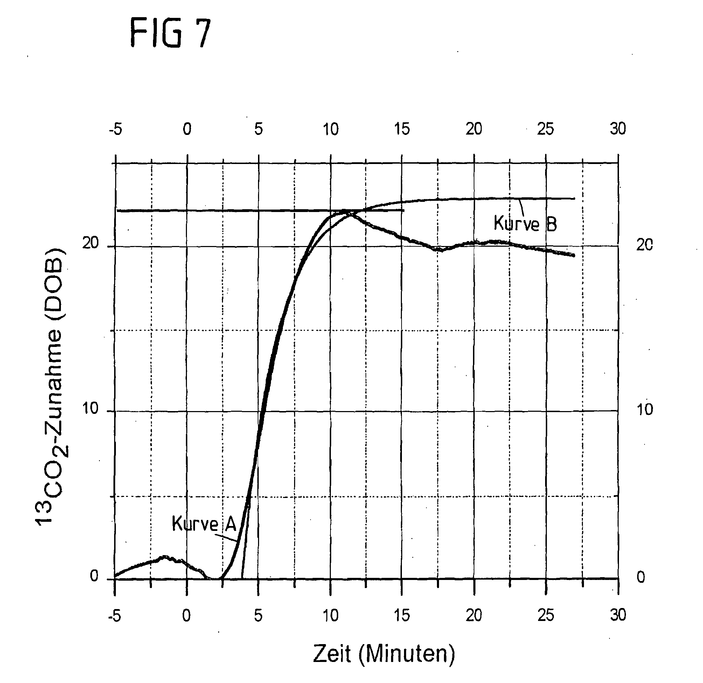

Figur 7 zeigt anhand eines Beispieles die Bestimmung der relevanten Parameter auf der Grundlage einer Kurve, die die Zunahme des $^{13}CO_2$ in der Atemluft nach Einnahme von $^{13}$C-markiertem Methacetin darstellt, vgl. hierzu auch die Erläuterung zur Figur 3. Basierend auf den ermittelten Datenpunkten (Kurve A) mit einem gemessenen maximalen Wert A von 22,01 DOB wird eine Anpassung (Fittung) mit einer wie oben beschriebenen Lösung einer Differentialgleichung erster Ordnung (Kurve B) durchgeführt. Aus der Lösung der Differentialgleichung gemäß

$$ y(t) = A_{max} - A_0 \exp(-\frac{t - t_0}{tau}) $$

erfolgt die Bestimmung der Amplitude $A_{max}$ der gefitteten Funktion mit 22,09 DOB und einer Zeitkonstante tau für die Umsetzung von 2,42 Minuten. Eine kleine Zeitkonstante von 2,42 Minuten weist dabei auf eine gute Leberpermeabilität hin, während ein langsamer Anstieg der auf den Messpunkten basierenden Kurve auf Zeitkonstanten im Bereich von über 5 Minuten und damit auf eine Verhärtung des Lebergewebes und dem damit verbundenen verschlechterte Leberpermeabilität hindeutet.

Neben oder zusätzlich der Bestimmung des Gehaltes eines $^{13}$C-markierten Metabolisierungsproduktes wie z.B. $^{13}CO_2$ in der Ausatemluft zur Einschätzung der Leberleistung ist es auch vorstellbar, die Konzentrationsabnahme des dealkylierten Produktes im Blut zu verfolgen und aus der entsprechenden Anstiegskinetik eine Zeitkonstante tau abzuleiten.

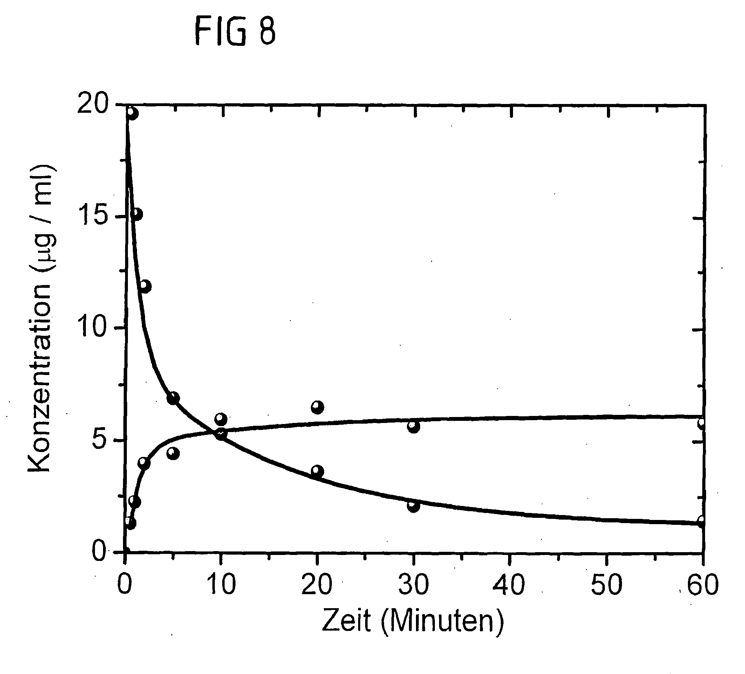

Diese Variante ist in Figur 8 gezeigt. Die Konzentrationsänderungen des verabreichten $^{13}$C-markierten Substrates z.B. $^{13}$C-Methacetin und des in der Leber gebildeten Dealkylierungsproduktes z.B. Paracetamol werden mittels eines geeigneten analytischen Verfahrens z.B. HPLC verfolgt. Die Konzentration des $^{13}$C-Methacetins nimmt aufgrund der Metabolsierung ab (exponentiell abnehmende Kurve beginnt bei einer Anfangskonzentration von 20 μg/ml $^{13}$C-Methacetin), während die Konzentration des Paracetamols im Gegenzug zunimmt (untere Kurve in Figur 8). Die anfänglichen Konzentrationsänderungen können auch hier mit einer Differentialgleichung erster Ordnung beschrieben werden. Mittels der beschrieben Lösung für eine Differentialgleichung erster Ordnung sind die entsprechenden Zeitkonstanten ableitbar, wobei die Zeitkonstante $\tau 1$ für die anfängliche rasche Konzentrationszunahme des Paracetamols 1,3 min beträgt, während die Zeitkonstante $\tau_2$ für die anschließende verlangsamte Konzentrationszunahme bedingt durch eine weitere Verteilung im Blut bei 16 min liegt.

Das vorliegende Verfahren zur Bereitstellung von Ausgangsdaten Bestimmung der Leberleistung ist für eine Vielzahl von Anwendungen einsetzbar.

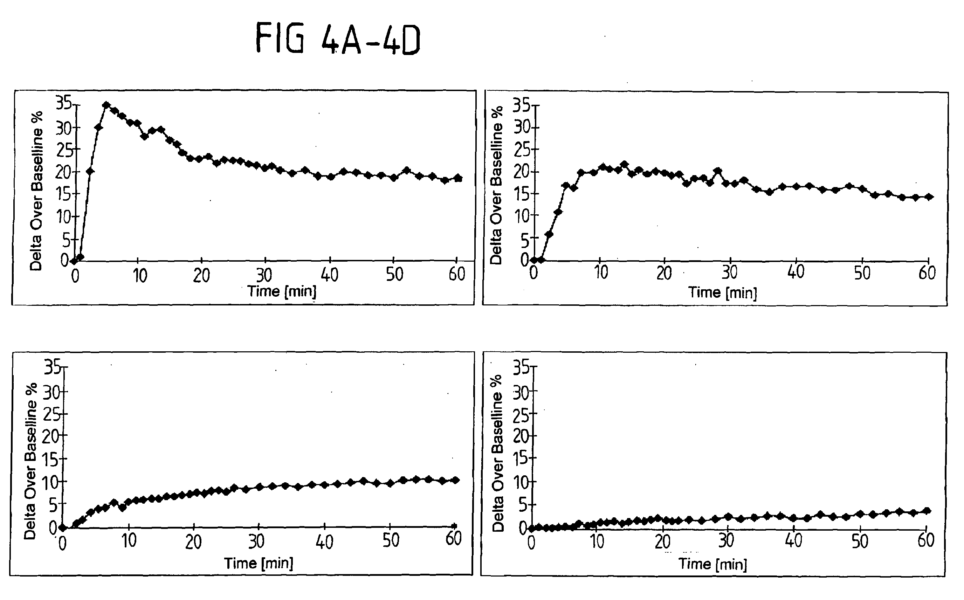

So erlaubt das Verfahren die Einschätzung des allgemeinen Gesundheitszustandes eines Patienten, insbesondere eine Einschätzung der Leberleistung eines Patienten. In den Figur 4a -d ist der Anstieg der Metabolisierung als Funktion der Zeit aufgetragen. Dabei ergeben sich für verschiedene Krankheitsbilder verschiedene Anstiegskinetiken mit unterschiedlichen Maximalwerten A und unterschiedlichen Zeitkonstanten $\tau$. Wie beschrieben, erlaubt der Wert A die Bestimmung des maximalen Umsatzes LiMAx, der direkt proportional zur Leberleistung ist. Figur 4a zeigt eine normale Leberleistung mit einem maximalen Umsatz LiMAx von 504 $\mu$g/h/kg, während in den Figuren 4b - 4d verschiedene Krankheitsbilder verdeutlicht werden. Bei einer Leberzirrhose ist die Metabolisierung des verabreichten Substrates eingeschränkt, so dass der maximale Umsatz LiMAx nur noch einen Wert von 307 $\mu$g/h/kg erreicht. Bei weiteren Leberschäden bis hin zum Leberversagen verringert sich der maximale Umsatz des verabreichten Substrates entsprechend auf einen Wert von 144 $\mu$g/h/kg (Figur 4c) bzw. 55 $\mu$g/h/kg (Figur 4d).

Das vorliegende Verfahren ermöglicht ebenfalls die Vorhersage bzw. Verfolgung der Lebergeneration und Überprüfung des Leberzustandes nach einer Operation wie z. B. nach einer Leberresektion. So ist es mittels dem vorliegenden Verfahren möglich, schon wenige Minuten nach einer Leberoperation oder sogar bereits während der Operation zu überprüfen, ob und in welchem Umfang die Leber leistungsfähig ist.

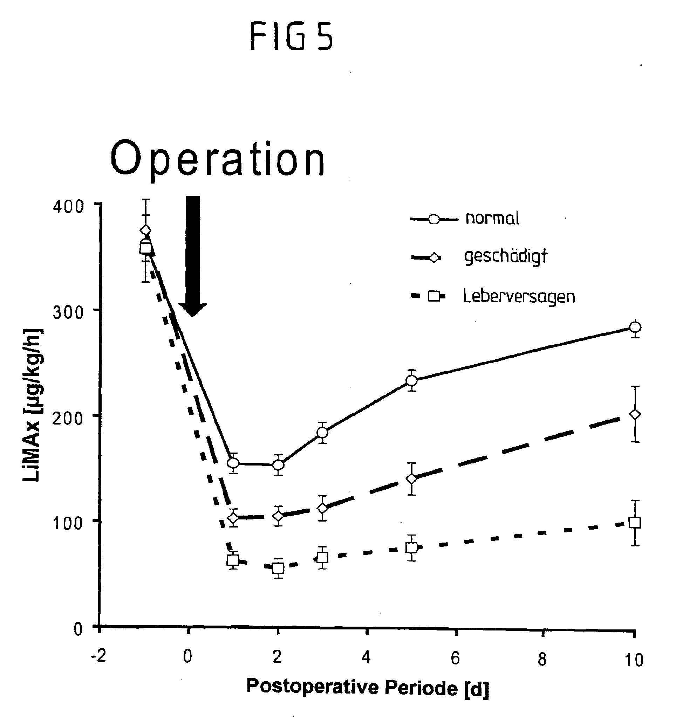

In Figur 5 sind die Leberleistungen nach einer Leberoperation dargestellt. So unterscheiden sich die maximalen Umsätze LiMAx signifikant zwischen einer gesunden regulären Leber, einer geschwächten Leber oder einer stark geschädigten Leber. Nach einer Operation dauert es in der Regel einige Tage, bis die Leber regeneriert. Ist der maximale Umsatz LiMAx und damit die Leberleistung nach einer Operation bereits sehr gering, kann vorhergesagt werden, dass die Leber des Patienten nicht gesunden wird und dieser mit hoher Wahrscheinlichkeit verstirbt. Durch das vorliegende Verfahren jedoch ist eine schnelle Erkennung solcher kritischen Fälle möglich, so dass die betroffenen Patienten alternativ z. B. durch eine Lebertransplantation behandelt werden können und dadurch gerettet werden.

Das vorliegende Verfahren ermöglicht ebenfalls eine Vorhersage des Operationsausganges vor einer Operation und somit eine geeignete Operationsplanung. So kann z. B. in Kombination mit einer CT-Volumetrie vor einer Leberoperation nicht nur das schadhafte Gewebe, wie z. B. Tumorgewebe, sondern auch das insgesamt notwendigerweise zu entfernende Gewebe bestimmt werden. Dies ist notwendig, da bei einer Tumorbehandlung möglichst viel Gewebe um den Tumor herum entfernt werden muss, um das Risiko des Streuens eines Tumors zu minimieren. Wird dabei jedoch zuviel Lebervolumen entfernt, besteht die Möglichkeit, dass der Patient stirbt. Die Größe des zu entfernenden Lebervolumens hängt von der Leberleistung des verbleibenden Lebervolumens ab. Durch die genaue Bestimmung der Leberleistung des vorhandenen Lebervolumens kann eine Operation daher punktgenau geplant werden, so dass der Patient optimale Überlebens- und Genesungschancen hat.

Dies sei anhand des folgenden Beispieles dargelegt. Beträgt das Tumorvolumen z. B. 153 ml, so ist es sinnvoll, insgesamt cirka 599 ml Lebervolumen zu entfernen. Bei einem Gesamtlebervolumen von 1.450 ml würde somit ein Restvolumen von 698 ml verbleiben, was ein Überleben des Patienten gewährleisten würde. Der maximale Umsatz LiMAx des verabreichten $^{13}$C-Methacetins beträgt vor der Operation 307 $\mu$g/h/kg. Das angestrebte Restvolumen von 698 ml würde einem maximalen Umsatz LiMAx von 165 $\mu$g/h/kg entsprechen. Bereits während der Operation kann daher mittels des vorliegenden Verfahrens der Umsatz kontinuierlich bestimmt werden, so dass gewährleistet wird, dass das für das Überleben notwendige Restvolumen von 698 ml erreicht wird. Im vorliegenden Fall beträgt das Restvolumen der Leber nach der Operation 625 ml und weist einen maximalen Umsatz von 169 $\mu$g/h/kg auf. Durch einen direkten Vergleich des gesunden Lebervolumens mit dem LiMAx-Wert kann das zu resizierende Lebervolumen über Dreisatz bestimmt werden um einen Ziel-LiMAx-Wert zu erreichen.

Das vorliegende Verfahren ermöglicht auch die Bestimmung der Funktion oder der postoperativen Nichtfunktion (PNS) einer transplantierten Leber. Nach einer Lebertransplantation kommt es in etwa 5 % der Fälle vor, dass die transplantierte Leber z. B. aufgrund einer unzureichenden Durchblutung nicht funktioniert. Bisher kann dies erst nach mehreren Tagen erkannt werden. Mit dem vorliegenden Verfahren ist es hingegen möglich, den Funktionsausfall der Leber schon nach wenigen Minuten zu erkennen, da die Zeitkonstante $\tau$ Informationen über die Zugänglichkeit des verabreichten Substrates in die Leber gibt. Entsprechend kann der Patient sofort behandelt werden und z. B. eine neue Transplantation vorgenommen werden.

Auch sind die Messung des Operationserfolges nach einer Lebertransplantation und die Planung von weiteren Behandlungsschritten mittels des vorliegenden Verfahrens möglich. So kann nach einer Lebertransplantation unmittelbar und direkt die Leistung der Leber mit dem vorliegenden Messverfahren bestimmt werden und die weitere Behandlung des Patienten individuell optimiert werden.

Das vorliegende Verfahren ermöglicht darüber hinaus die Einschätzung einer Sepsisgefahr für Intensivpatienten. Es ist bekannt, dass in der Intensivmedizin die Gefahr an Sepsis zu versterben sehr hoch ist. Mit dem vorliegenden Messverfahren ist es nunmehr möglich, direkt bei Einlieferung und während der Behandlung eine Leberschädigung oder eine normale Funktion der Leberzellen zu bestimmen.

Die Bestimmung der Leberschädigung ist auch insbesondere bei der Zulassung von Medikamenten und Arzneimitteln von Bedeutung. Daher ist eine der wichtigsten Anwendungsgebiete des vorliegenden Verfahrens der Einsatz des Verfahrens zur Überprüfung von Leberschädigungen hervorgerufen durch Medikamente und Arzneimittel im Verlaufe einer Arzneimittelzulassung. Im Rahmen der Arzneimittelzulassung muss in einer Toxikologieprüfung gezeigt werden, dass die zur Zulassung stehenden Arzneimittel nicht die Leber schädigen. Üblicherweise wird eine sichere Risikoabschätzung aus einer Reihe von verschiedenen Tierversuchen abgeleitet. Dennoch treten bei Menschen oftmals unerwartete Nebenwirkungen auf, die im Rahmen der Tierversuche nur schwer nachweisbar sind. Mit dem vorliegenden Verfahren hingegen kann eine toxische Wirkung auf Tier und Mensch exakt und quantitativ ermittelt werden. Aufgrund des vorliegenden Verfahrens, das eine zuverlässige quantitative Bestimmung der Leberleistung zulässt, ist es nunmehr möglich, Untersuchungen zur Arzneimitteldosierung schneller und exakter durchzuführen.

Auch können Langzeitschäden verbunden mit einem Umbau der Leber hervorgerufen durch Arzneimittel, wie z. B. Kontrazeptiva, mittels des vorliegenden Verfahrens verfolgt werden. Bei der regelmäßigen Einnahme von Arzneimitteln, wie z. B. von Kontrazeptiva, können Veränderungen der Leber auftreten, die zuerst die Zugänglichkeit der Leberzellen beeinflussen und später zu einer Reduktion der Leberleistung führen. Diese Veränderungen der Leber können durch die Anstiegszeiten $\tau$, über die die Eintrittsgeschwindigkeit der Substanzen in die Leberzellen bestimmbar ist, und der Maximalwert A, der Aussagen über die Anzahl der gesunden Leberzellen ermöglicht, nachgewiesen werden. Regelmäßige Tests mit dem vorliegenden Messverfahren ermöglichen daher die Anzeige solcher Leberänderungen. Basierend auf den ermittelten Daten kann der Arzt eine Änderung der Arzneimittelvergabe durchführen, so dass keine weiteren Leberänderungen stattfinden.

Der Einfluss von genetisch veränderten Stoffen und Lebensmitteln auf Lebewesen, insbesondere den Menschen, ist derzeit nur schwer nachweisbar. Dies ist insbesondere dadurch bedingt, dass die Konzentrationen schädlicher biologischer Stoffe oft unter oder knapp an der Nachweisgrenze liegen oder aber deren Schädlichkeit bisher überhaupt nicht bekannt ist. Das vorliegende Verfahren ermöglicht einen eindeutigen Nachweis der Schädigung der Leber durch genetisch veränderte Lebensmittel.

Ebenfalls können mit dem vorliegenden Verfahren Einflüsse von Chemikalien in der chemischen Industrie oder der Pharmaindustrie verfolgt, überwacht und identifiziert werden. Das ermöglicht eine zuverlässige Überprüfung der Gesundheit der Menschen am Arbeitsplatz.

Weitere Anwendungsgebiete des vorliegenden Verfahrens liegen auf den Gebieten der Arbeitsmedizin zum Abschätzen von Krankheitsrisiken, in der Leberkrebsvorsorge, bei der Überwachung von Leberkrankheiten, wie z. B. Hepatitis, beim Nachweis von Leberschäden bei Tieren, wie z. B. hervorgerufen durch das Jacobkreuzkraut bei Pferden, Vergiftungen und in der Umweltmedizin zur Suche nach leberschädlichen Substanzen im Boden, Nahrung und/oder im Trinkwasser.

Ein besonders bevorzugtes Einsatzgebiet des vorliegenden Verfahrens ist das Einstellen von Medikamenten. Da die Leber die Mehrzahl aller verabreichten Arzneimittel metabolisiert, wird bei einer hohen Leberleistung entsprechend ein Großteil der Arzneimittel metabolisiert, während bei einer schlechteren Leberleistung ein geringerer Anteil der Arzneimittel metabolisiert wird. Das bedeutet jedoch für einen Patienten, dass je nach Leberleistung die Dosierung des Arzneimittels im Körper unterschiedlich hoch ist und somit auch eine unterschiedliche Wirksamkeit entfalten kann. Daher sollte eine optimale Wirkung der Arzneimittel auf die Leberleistung angepasst sein. Als Beispiel sei hier auf die Vergabe von Tacrolimus, einem Immunosupressivum gegen Abstoßreaktionen bei einer Organtransplantation hingewiesen. Die exakte Einstellung einer Dosis Tacrolimus ist von großer Bedeutung, da eine zu hohe Dosis Tacrolimus toxisch wirkt und eine zu geringe Dosis wirkungslos ist. Bei genauer Kenntnis der Leberleistung ist es nunmehr möglich, die Dosis exakt einzustellen und die Wirkung des Arzneimittels zu optimieren.

Das vorliegende Verfahren kann ebenfalls aufgrund seiner Einfachheit und Schnelligkeit bei einem Hausarzt zum Zwecke eines Leberchecks verwendet werden, um die Leberleistung als Teil des Gesundheitszustandes abzufragen.

**Patentansprüche**

1. Verfahren zur Bereitstellung von Ausgangsdaten zur Bestimmung der Leberleistung eines Lebewesens, insbesondere eines Menschen, umfassend

    - die Bestimmung des Gehaltes von $^{13}CO_2$ als Metabolisierungsprodukt eines $^{13}C$-markierten Substrates in der Ausatemluft eines Lebewesens über ein bestimmtes Zeitintervall mittels mindestens eines Messgerätes mit mindestens einer Auswerteeinheit, wobei das Substrat dem Lebewesen zuvor verabreicht wurde und durch die Leber unter Freisetzung des $^{13}CO_2$ umgesetzt wird,

    **dadurch gekennzeichnet, dass**
    die gemessene anfängliche Zunahme des Gehaltes des $^{13}CO_2$ in der Ausatemluft mit einer Differentialgleichung erster Ordnung beschrieben wird und aus der Lösung der Differentialgleichung erster Ordnung der Wert $A_{max}$ für die maximale Konzentration des $^{13}CO_2$ und die Zeitkonstante tau der Zunahme des Gehaltes des $^{13}CO_2$ bestimmt werden, wobei als Lösung der Differentialgleichung erster Ordnung die Gleichung

$$y(t) = A_{\max} - A_0 \exp(-\frac{t - t_0}{tau})$$

verwendet wird, wobei y(t) die Metabolisierungsdynamik des mindestens einen Substrates, $A_{\max}$ die maximale Amplitude der gefitteten Funktion bzw. die maximale Konzentration des $^{13}CO_2$, $A_0$ die Anfangskonzentration des $^{13}CO_2$, tau die Zeitkonstante, to den Beginn der Metabolisierung und t die Messzeit darstellt.

2. Verfahren nach Anspruch 1, **dadurch gekennzeichnet, dass** die $^{13}CO_2$-Zunahme in der Ausatemluft bis zu einem Wert von 70 % des Maximalwerts des $^{13}CO_2$, insbesondere bis zum Maximalwert des $^{13}CO_2$, mit einer Differentialgleichung erster Ordnung beschrieben wird.

3. Verfahren nach einem der vorhergehenden Ansprüche, **dadurch gekennzeichnet, dass** die Menge des gebildeten $^{13}CO_2$ proportional zu der Menge des mindestens einen verabreichten Substrates ist.

4. Verfahren nach Anspruch 1, **dadurch gekennzeichnet, dass** die genannte Exponentialfunktion an die Messwerte der anfänglichen Zunahme des Gehaltes des $^{13}CO_2$ in der Ausatemluft angepasst wird und aus der Anpassung der maximale Wert $A_{\max}$ und die Zeitkonstante tau bestimmt werden.

5. Verfahren nach einem der vorhergehenden Ansprüche, **dadurch gekennzeichnet, dass** aus dem Wert $A_{\max}$ der maximale Umsatz des mindestens einen Substrates in der Leber durch die Gleichung

$$LiMAx = \frac{A_{\max} R_{PBD} PM}{BW}$$

bestimmt wird, wobei $R_{PBD}$ als Pee-Dee-Belemnite-Standard des $^{13}CO_2/^{12}CO_2$-Verhältnisses dem Wert 0,011237, P der $CO_2$-Produktionsrate, M der molaren Masse der verabreichten Substanz und BW dem Körpergewicht der Person entsprechen.

6. Verfahren nach einem der vorhergehenden Ansprüche, **dadurch gekennzeichnet, dass** als $^{13}C$-markiertes Substrat ein Substrat verwendet wird, aus welchem $^{13}CO_2$ mittels einer Dealkylierungsreaktion einer Alkoxygruppe, insbesondere einer Methoxygruppe, freigesetzt wird.

7. Verfahren nach einem der vorhergehenden Ansprüche, **dadurch gekennzeichnet, dass** der absolute Gehalt des $^{13}CO_2$ in der Ausatemluft bestimmt wird.

8. Verfahren nach einem der vorhergehenden Ansprüche, **dadurch gekennzeichnet, dass** die Bestimmung des gebildeten $^{13}CO_2$ in Echtzeit erfolgt.

9. Verfahren nach einem der vorhergehenden Ansprüche, **dadurch gekennzeichnet, dass** der Gehalt des gebildeten $^{13}CO_2$ in der Ausatemluft kontinuierlich im Messgerät bestimmt wird.

10. Verfahren nach einem der vorhergehenden Ansprüche, **dadurch gekennzeichnet, dass** es mit der Magnetresonanztomographie als einem weiteren analytischen Verfahren derart kombiniert wird, dass die Metabolisierungsdynamik durch ein Verfahren nach einem der vorhergehenden Ansprüche bestimmt wird und mit zeitaufgelöster Magnetresonanztomographie verglichen wird, um eine räumliche und zeitliche Auflösung der Metabolisierung einzelner Enzyme zu untersuchen.

**Claims**

1. Method for providing original data for determining the liver performance of a living organism, in particular a human, comprising

  - determining the amount of $^{13}CO_2$ as metabolization product of a $^{13}C$ labelled substrate product in the exhalation air over a definite time interval by the means of at least one measuring device with at least one evaluation unit, wherein the substrate was administered previously to the living organism and converted by the liver by releasing $^{13}CO_2$.

 **characterized in that**
 the measured initial increase of the amount of $^{13}CO_2$ in the exhalation air is described by a differential equation of first order and the value $A_{\max}$ for the maximum concentration of the $^{13}CO_2$ and the time constant tau of the increase of the amount of $^{13}CO_2$ are determined from the solution of the differential equation of first order, wherein as solution of the differential equation of first order the equation

$$y(t) = A_{\max} - A_0 \exp(-\frac{t - t_0}{tau})$$

 is used, wherein y(t) describes the metabolization dynamics of the at least one substrate, $A_{\max}$ the maximum amplitude of the fitted function or the maximum concentration of the $^{13}CO_2$, $A_0$ the initial concentra-

tion of the $^{13}CO_2$, tau the time constant, to the start of the metabolization and t the measuring time.

2. Method according to claim 1, **characterized in that** the $^{13}CO_2$ increase in the exhalation air is described up to a value of 70% of the maximum value of the $^{13}CO_2$, in particular up to the maximum value of the $^{13}CO_2$ by a differential equation of first order.

3. Method according to any of the preceding claims, **characterized in that** the amount of the formed $^{13}CO_2$ is proportional to the amount of the at least one administered substrate.

4. Method according to claim 1, **characterized in that** the said exponential function is adapted to the measured data of the initial increase of the amount of the $^{13}CO_2$ in the exhalation air and the maximum value $A_{max}$ and the time constant tau are determined from the adaptation.

5. Method according to any of the preceding claims, **characterized in that** based on the value $A_{max}$ the maximum conversion of the at least one substrate in the liver is determined by the equation

$$LiMAx = \frac{A_{max} R_{PBD} PM}{BW}$$

wherein $R_{PBD}$ as the Pee-Dee-Belemnite-Standard of the $^{13}CO/^2CO_2$-ratio corresponds to the value 0,011237, P to the $CO_2$ production rate, M to the molar mass of the administered substance and BW to the body weight of the person.

6. Method according to any of the preceding claims, **characterized in that** a substrate is used as $^{13}C$ labelled substrate from which $^{13}CO_2$ is released by the means of a dealkylation reaction of an alkoxy group, in particular a methoxy group.

7. Method according to any of the preceding claims, **characterized in that** the absolute $^{13}CO_2$ amount in the exhalation air is determined.

8. Method according to any of the preceding claims, **characterized in that** the determination of the formed $^{13}CO_2$ occurs in real time.

9. Method according to any of the preceding claims, **characterized in that** the amount of the formed $^{13}CO_2$ in the exhalation air is continuously determined by the measuring device.

10. Method according to any of the preceding claims, **characterized in that** said method is combined to magnetic resonance imaging as a further analytical method such that the metabolization dynamic is determined by a method according to one of the preceding claims and is compared to time resolved magnetic resonance imaging in order to investigate a spatial and timely resolution of the metabolization of singular enzymes.

**Revendications**

1. Procédé pour la mise à disposition de données de départ pour la détermination de la fonction hépatique d'un être vivant, en particulier d'un être humain, comprenant

- la détermination de la teneur en $^{13}CO_2$ comme produit de métabolisation d'un substrat marqué par $^{13}C$ dans l'air expiré d'un être vivant sur un intervalle de temps déterminé au moyen d'au moins un appareil de mesure à l'aide d'au moins une unité d'évaluation, le substrat ayant été administré au préalable à l'être vivant et étant transformé par le foie avec libération du $^{13}CO_2$,

**caractérisé en ce que** l'augmentation initiale mesurée de la teneur en $^{13}CO_2$ dans l'air expiré est décrite par une équation différentielle de premier ordre et on détermine à partir de la solution de l'équation différentielle de premier ordre la valeur $A_{max}$ pour la concentration maximale en $^{13}CO_2$ et la constante de temps tau de l'augmentation de la teneur en $^{13}CO_2$, en utilisant comme solution de l'équation différentielle de premier ordre l'équation

$$y(t) = A_{max} - A_0 \exp(-\frac{t - t_0}{tau})$$

où y(t) représente la dynamique de métabolisation dudit au moins un substrat, $A_{max}$ représente l'amplitude maximale de la fonction ajustée ou, selon le cas, la concentration maximale en $^{13}CO_2$, $A_0$ représente la concentration de départ en $^{13}CO_2$, tau représente la constante de temps, $t_0$ représente le début de la métabolisation et t représente le temps de mesure.

2. Procédé selon la revendication 1, **caractérisé en ce que** l'augmentation en $^{13}CO_2$ dans l'air expiré est décrite jusqu'à une valeur représentant 70% de la valeur maximale de $^{13}CO_2$, en particulier jusqu'à la valeur maximale de $^{13}CO_2$, par une équation différentielle de premier ordre.

3. Procédé selon l'une quelconque des revendications précédentes, **caractérisé en ce que** la quantité du $^{13}CO_2$ formé est proportionnelle à la quantité dudit au moins un substrat administré.

4. Procédé selon la revendication 1, **caractérisé en ce que** la fonction exponentielle mentionnée est adaptée aux valeurs de mesure de l'augmentation initiale de la teneur en $^{13}CO_2$ dans l'air expiré et on détermine, à partir de l'adaptation, la valeur maximale $A_{max}$ et la constante de temps tau.

5. Procédé selon l'une quelconque des revendications précédentes, **caractérisé en ce qu'**on détermine, à partir de la valeur $A_{max}$, la conversion maximale dudit au moins un substrat dans le foie par l'équation

$$LiMAx = \frac{A_{max} R_{PBD} PM}{BW}$$

où $R_{PBD}$, en tant que standard Pee-Dee-Belemnite du rapport $^{13}CO_2/^{12}CO_2$, correspond à la valeur 0,011237, P correspond au taux de production de $CO_2$, M correspond à la masse molaire de la substance administrée et BW correspond au poids corporel de la personne.

6. Procédé selon l'une quelconque des revendications précédentes, **caractérisé en ce qu'**on utilise comme substrat marqué par $^{13}C$ un substrat à partir duquel du $^{13}CO_2$ est libéré au moyen d'une réaction de désalkylation d'un groupe alcoxy, en particulier d'un groupe méthoxy.

7. Procédé selon l'une quelconque des revendications précédentes, **caractérisé en ce qu'**on détermine la teneur absolue en $^{13}CO_2$ dans l'air expiré.

8. Procédé selon l'une quelconque des revendications précédentes, **caractérisé en ce que** la détermination du $^{13}CO_2$ formé a lieu en temps réel.

9. Procédé selon l'une quelconque des revendications précédentes, **caractérisé en ce que** la teneur en $^{13}CO_2$ formé dans l'air expiré est déterminée en continu dans l'appareil de mesure.

10. Procédé selon l'une quelconque des revendications précédentes, **caractérisé en ce qu'**il est combiné avec la tomographie par résonance magnétique comme autre procédé analytique de manière telle que la dynamique de métabolisation est déterminée selon un procédé selon l'une quelconque des revendications précédentes et comparée à une tomographie par résonance magnétique résolue dans le temps pour étudier une résolution dans l'espace et dans le temps de la métabolisation de certaines enzymes.

EP 2 365 777 B1

# FIG 1

# FIG 2

# FIG 3

FIG 4A-4D

FIG 5

# FIG 6

FIG 7

## FIG 8

EP 2 365 777 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007000145 A2

- WO 2007107366 A1

**In der Beschreibung aufgeführte Nicht-Patentliteratur**

- **VON MATSUMOTO et al.** *Digestive Diseases Science,* 1987, vol. 32, 344-348
- **BRADEN et al.** *Aliment Pharmacol. Ther.,* 2005, vol. 21, 179-185

- **STOCKMANN et al.** Annals of Surgery. 2009, vol. 250, 119-125